# EUROPEAN PATENT SPECIFICATION

(11) **EP 0 872 239 B1**
(45) Date of publication and mention of the grant of the patent: **13.06.2001**
(21) Application number: 98302794.7
(22) Date of filing: 09.04.1998
(51) Int. Cl.: A61K 31/55

(54) **Use of olanzapine for the manufacture of a medicament for the treatment of cerebral focal ischemia**
Verwendung von Olanzapine zur Herstellung eines Arzneimittels zur Behandlung der zerebralen fokalen Ischämie
Utilisation de l'olanzapine pour la fabrication d'un médicament pour le traitement de l'ischémie cérébrale focale

(30) Priority: 15.04.1997 US 43095 P
(43) Date of publication of application: 21.10.1998
(73) Proprietor: ELI LILLY AND COMPANY, Indianapolis, Indiana 46285 (US)
(72) Inventor: Bymaster, Franklin Porter, Brownsburg, Indiana 46112 (US); Tollefson, Gary Dennis, Indianapolis, Indiana 46236 (US)
(74) Representative: Hudson, Christopher Mark

(56) References cited:
- EP-A- 0 733 635

## Description

This invention provides the use of 2-methyl-4-(4-methyl-1-piperazinyl)-10H-thieno[2,3-b] [1,5] benzodiazepine, (hereinafter referred as "olanzapine" for the manufacture of a medicament for the treatment or inhibition of cerebral focal stroke.

Stroke remains the third most common cause of death in the industrial world, ranking behind ischemic heart disease and cancer. Strokes are responsible for about 300,000 deaths annually in the United States and are a leading cause of hospital admissions and long-term disabilities. Accordingly, the socioeconomic impact of stroke and its attendant burden on society is practically immeasurable.

A significant event in cerebral ischemia is known as the transient ischemic attack ("TIA"). A TIA is defined as a neurologic deficit with a duration of less than 24 hours. The TIA is an important sign of an ischemic development that may lead to cerebral infarction.
Presently, no ideal treatment for TIA exists, and there are no generally accepted guidelines as to whether medical or surgical procedures should be carried out in order to reduce the incidence of stroke in subjects with TIA.

The etiology of TIA involves hemodynamic events and thromboembolic mechanisms. Because most TIAs resolve within one hour, a deficit that lasts longer is often classified as presumptive stroke and is, accordingly, associated with permanent brain injury. Therefore, computed tomographic brain scans are used to search for cerebral infarction in areas affected by TIAs lasting longer than several hours. The relevant clinical distinction between a TIA and a stroke is whether the ischemia has caused brain damage, which is typically classified as infarction or ischemic necrosis. Subjects with deteriorating clinical signs might have stroke in evolution or are classified as having progressive stroke. In this clinical setting, clot propagation is possibly an important factor in disease progression.

At present, the pharmacological therapy of cerebral focal stroke is limited to symptomatic treatments that do not alter the course of the underlying disease.

Meanwhile, because of the current dissatisfaction with the currently marketed treatments for the above-described indications within the affected population, the need continues for safer, better-calibrated drugs which could be used to treat or inhibit cerebral focal stroke.

The present invention provides a compound useful for treating and/or inhibiting cerebral focal stroke.

This invention provides the use of olanzapine or a pharmaceutically acceptable salt or solvate thereof, for the manufacture of a medicament for the treatment or inhibition of cerebral focal stroke in mammals.

Olanzapine is of the formula 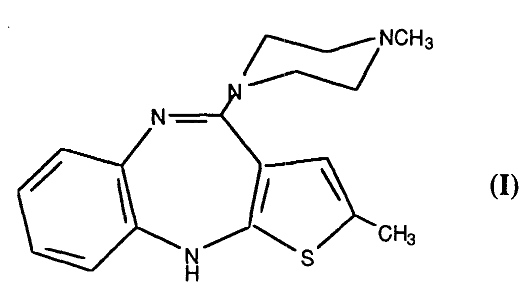 or an acid addition salt thereof.

It is especially preferred that olanzapine will be the Form II olanzapine polymorph having a typical x-ray powder diffraction pattern as represented by the following interplanar spacings:

| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

A typical example of an x-ray diffraction pattern for Form II is as follows wherein d represents the interplanar spacing and I/I₁ represents the typical relative intensities:

| **d** | **I/I**_{**1**} |
|---|---|
| 10.2689 | 100.00 |
| 8.577 | 7.96 |
| 7.4721 | 1.41 |
| 7.125 | 6.50 |
| 6.1459 | 3.12 |
| 6.071 | 5.12 |
| 5.4849 | 0.52 |
| 5.2181 | 6.86 |
| 5.1251 | 2.47 |
| 4.9874 | 7.41 |
| 4.7665 | 4.03 |
| 4.7158 | 6.80 |
| 4.4787 | 14.72 |
| 4.3307 | 1.48 |
| 4.2294 | 23.19 |
| 4.141 | 11.28 |
| 3.9873 | 9.01 |
| 3.7206 | 14.04 |
| 3.5645 | 2.27 |
| 3.5366 | 4.85 |
| 3.3828 | 3.47 |
| 3.2516 | 1.25 |
| 3.134 | 0.81 |
| 3.0848 | 0.45 |
| 3.0638 | 1.34 |
| 3.0111 | 3.51 |
| 2.8739 | 0.79 |
| 2.8102 | 1.47 |
| 2.7217 | 0.20 |
| 2.6432 | 1.26 |
| 2.6007 | 0.77 |

The x-ray diffraction patterns set out herein were obtained using a Siemens D5000 x-ray powder diffractometer having a copper Ka radiation source of wavelength, 1 =1·541Å.

It is further preferred that the Form II olanzapine polymorph will be administered as the substantially pure Form II olanzapine polymorph.

As used herein "substantially pure" refers to Form II associated with less than about 5% Form I, preferably less than about 2% Form I, and more preferably less than about 1% Form I. Further, "substantially pure" Form II will contain less than about 0.5% related substances, wherein "related substances" refers to undesired chemical impurities or residual solvent or water. In particular, "substantially pure" Form II should contain less than about 0.05% content of acetonitrile, more preferably, less than about 0.005% content of acetonitrile. Additionally, the polymorph of the invention should contain less than 0.5% of associated water.

The polymorph obtainable by the process taught in the '382 patent will be designated as Form I and has a typical x-ray powder diffraction pattern substantially as follows, obtained using a Siemens D5000 x-ray powder diffractometer, wherein d represents the interplanar spacing:

| **d** |
|---|
| 9.9463 |
| 8.5579 |
| 8.2445 |
| 6.8862 |
| 6.3787 |
| 6.2439 |
| 5.5895 |
| 5.3055 |
| 4.9815 |
| 4.8333 |
| 4.7255 |
| 4.6286 |
| 4.533 |
| 4.4624 |
| 4.2915 |
| 4.2346 |
| 4.0855 |
| 3.8254 |
| 3.7489 |
| 3.6983 |
| 3.5817 |
| 3.5064 |
| 3.3392 |
| 3.2806 |
| 3.2138 |
| 3.1118 |
| 3.0507 |
| 2.948 |
| 2.8172 |
| 2.7589 |
| 2.6597 |
| 2.6336 |
| 2.5956 |

A typical example of an x-ray diffraction pattern for Form I is as follows wherein d represents the interplanar spacing and I/I₁ represents the typical relative intensities:

| **d** | **I/I** _{**1**} |
|---|---|
| 9.9463 | 100.00 |
| 8.5579 | 15.18 |
| 8.2445 | 1.96 |
| 6.8862 | 14.73 |
| 6.3787 | 4.25 |
| 6.2439 | 5.21 |
| 5.5895 | 1.10 |
| 5.3055 | 0.95 |
| 4.9815 | 6.14 |
| 4.8333 | 68.37 |
| 4.7255 | 21.88 |
| 4.6286 | 3.82 |
| 4.533 | 17.83 |
| 4.4624 | 5.02 |
| 4.2915 | 9.19 |
| 4.2346 | 18.88 |
| 4.0855 | 17.29 |
| 3.8254 | 6.49 |
| 3.7489 | 10.64 |
| 3.6983 | 14.65 |
| 3.5817 | 3.04 |
| 3.5064 | 9.23 |
| 3.3392 | 4.67 |
| 3.2806 | 1.96 |
| 3.2138 | 2.52 |
| 3.1118 | 4.81 |
| 3.0507 | 1.96 |
| 2.948 | 2.40 |
| 2.8172 | 2.89 |
| 2.7589 | 2.27 |
| 2.6597 | 1.86 |
| 2.6336 | 1.10 |
| 2.5956 | 1.73 |

The x-ray powder diffraction patterns herein were obtained with a copper Ka of wavelength 1 = 1.541Å. The interplanar spacings in the column marked "d" are in Angstroms. The typical relative intensities are in the column marked "I/I₁".

As used herein, the term "mammal" shall refer to the Mammalia class of higher vertebrates. The term "mammal" includes, but is not limited to, a human. The term "treating" as used herein includes prophylaxis of the named condition or amelioration or elimination of the condition once it has been established.

Olanzapine is effective over a wide dosage range; however, it is desirable to administer a dosage that is as low as possible. For example, dosages per day of the olanzapine will normally fall within the range of about 1 mg to about 30 mg per day. However, it will be understood that the amount of the compound actually administered will be determined by a physician, in the light of the relevant circumstances including the type of neuro-protective effect or condition to be treated, the age, weight, and response of the individual patient, the severity of the patient's symptoms, and the chosen route of administration, and therefore the above dosage ranges are not intended to limit the scope of the invention in any way. While the present compounds are preferably administered orally to humans susceptible to or suffering from cerebral focal stroke, the compounds may also be administered by a variety of other routes such as the transdermal, parenterally, subcutaneous, intranasal, intramuscular and intravenous routes. Such formulations may be designed to provide delayed or controlled release using formulation techniques which are known in the art.

As used herein the term "treating" includes prophylaxis of cerebral focal stroke in a patient having a tendency to develop such cerebral focal stroke, and the amelioration or elimination of the cerebral focal stroke once it has been established or alleviation of the characteristic symptoms of such cerebral focal stroke.

The results of pharmacological studies show that olanzapine has muscarinic cholinergic receptor activity. The compound is active at the dopamine D-1 and D-2 receptors as indicated by an IC50 of less than 1 uM in the 3H-SCH233390 (Billard, et al. Life Sciences **35:**1885 (1984)) and the 3H spiperone (Seeman et al Nature **216:**717 (1976)) binding assays respectively. Further, olanzapine is active at the 5-HT-2 receptor and 5-HT1C receptor. The complex pharmacological profile of the compound provides a medicament which can unexpectedly be useful for the treatment of cerebral focal stroke.

Compositions suitable for internal administration contain from one half (0.5) milligrams to 50 milligrams olanzapine per unit.

Typical compositions include olanzapine or a pharmaceutically acceptable acid addition salt thereof, associated with a pharmaceutically acceptable excipient which may be a carrier, or a diluent or be diluted by a carrier, or enclosed within a carrier which can be in the form of a capsule, sachet, paper, or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone. The formulations may also include wetting agents, emulsifying and suspending agents, preserving agents, sweetening agents, or flavoring agents. The formulations of t:he invention may be formulated so as to provide quick, sustained, or delayed release of the active ingredient after administration to the patient by employing procedures well known in the art.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

Generally, olanzapine is dispensed in unit form comprising from 1 mg to 30 mg in a pharmaceutically acceptable carrier per unit dosage.

Most preferably, the solid oral formulation is contained in packaging materials which protect the formulation from moisture and light. For example, suitable packaging materials include amber colored high density polyethylene bottles, amber colored glass bottles, and other containers made of a material which inhibits the passage of light. Most preferably, the packaging will include a desiccant pack. The container may be sealed with an aluminium foil blister to provide the desired protection and maintain product stability.

The compositions of this invention may be suitable for administration to an animal. Such animals include both domestic animals, for example livestock, laboratory animals, and household pets, and non-domestic animals such as wildlife. More preferably, the animal is a vertebrate. Most preferably, a compound of this invention shall be administered to a mammal. It is especially preferred that the animal is a domestic mammal or a human. For such purposes, a compound of this invention may be administered as a feed additive. The most preferred mammal is a human.

"Stroke" is defined by the World Health Organization as a rapidly developing clinical sign of focal or global disturbance of cerebral function with symptoms lasting at least 24 hours. Strokes are also implicated in deaths where there is no apparent cause other than an effect of vascular origin.

Strokes are typically caused by blockages or occlusions of the blood vessels to the brain or within the brain. With complete occlusion, arrest of cerebral circulation causes cessation of neuronal electrical activity within seconds. Within a few minutes after the deterioration of the energy state and ion homeostasis, depletion of high energy phosphates, membrane ion pump failure, efflux of cellular
potassium, influx of sodium chloride and water, and membrane depolarization occur. If the occlusion persists for more than five to ten minutes, irreversible damage results. With incomplete ischemia, however, the outcome is difficult to evaluate and depends largely on residual perfusion and the availability of oxygen. After a thrombotic occlusion of a cerebral vessel, ischemia is rarely total. Some residual perfusion usually persists in the ischemic area, depending on collateral blood flow and local perfusion pressure.

Cerebral blood flow can compensate for drops in mean arterial blood pressure from 90 to 60 mm Hg by autoregulation. This phenomenon involves dilatation of downstream resistant vessels. Below the lower level of autoregulation (about 60 mm Hg), vasodilatation is inadequate and the cerebral blood flow falls. The brain, however, has perfusion reserves that can compensate for the fall in cerebral blood fall. This reserve exists because under normal conditions only about 35% of the oxygen delivered by the blood is extracted. Therefore, increased oxygen extraction can take place, provided that normoxia and normocapnea exist. When distal blood pressure falls below approximately 30 mm Hg, the two compensatory mechanisms (autoregulation and perfusion reserve) are inadequate to prevent failure of oxygen delivery.

As flows drops below the ischemic threshold of 23 ml/100 g/minute, symptoms of tissue hypoxia develop. Severe ischemia may be lethal. When the ischemia is moderate, it will result in "penumbra." In the neurological context, penumbra refers to a zone of brain tissue with moderate ischemia and paralyzed neuronal function, which is reversible with restoration of adequate perfusion. The penumbra forms a zone of collaterally perfused tissue surrounding a core of severe ischemia in which an infarct has developed. In other words, the penumbra is the tissue area that can be saved, and is essentially in a state between life and death.

Although an ischemic event can occur anywhere in the vascular system, the carotid artery bifurcation and the origin of the internal carotid artery are the most frequent sites for thrombotic occlusions of cerebral blood vessels, which result in cerebral ischemia. The symptoms of reduced blood flow due to stenosis or thrombosis are similar to those caused by middle cerebral artery disease. Flow through the ophthalmic artery is often affected sufficiently to produce amaurosis fugax or transient monocular blindness. Severe bilateral internal carotid artery stenosis may result in cerebral hemispheric hypoperfusion. This manifests with acute headache ipsilateral to the acutely ischemic hemisphere. Occlusions or decrease of the blood flow with resulting ischemia of one anterior cerebral artery distal to the anterior communicating artery produces motor and cortical sensory symptoms in the contralateral leg and, less often, proximal arm. Other manifestations of occlusions or underperfusion of the anterior cerebral artery include gait ataxia and sometimes urinary incontinence due to damage to the parasagittal frontal lobe. Language disturbances manifested as decreased spontaneous speech may accompany generalized depression of psychomotor activity.

Most ischemic strokes involve portions or all of the territory of the middle cerebral artery with emboli from the heart or extracranial carotid arteries accounting for most cases. Emboli may occlude the main stem of the middle cerebral artery, but more frequently produce distal occlusion of either the superior or the inferior branch. Occlusions of the superior branch cause weakness and sensory loss that are greatest in the face and arm. Occlusions of the posterior cerebral artery distal to its penetrating branches cause complete contralateral loss of vision. Difficulty in reading (dyslexia) and in performing calculations (dyscalculia) may follow ischemia of the dominant posterior cerebral artery. Proximal occlusion of the posterior cerebral artery causes ischemia of the branches penetrating to calamic and limbic structures. The clinical results are hemisensory disturbances that may chronically change to intractable pain of the defective side (thalamic pain).

A basilar artery occlusion produces massive deficits. One of these deficits is known as the "locked in state." In this condition, paralysis of the limbs and most of the bulbar muscle leaves the subject only able to communicate by moving the eyes or eyelids in a type of code. Occlusion of the basilar apex or top of the basilar is usually caused by emboli that lodge at the junction between the basilar artery and the two posterior cerebral arteries. The condition produces an initial reduction in arousal followed by blindness and amnesia due to an interruption of flow into the posterior cerebral arteries as well as abnormalities of vertical gaze and pupillary reactivity due to tegmental damage.

Venous occlusion can cause massive damage and death. This disease is less common than arterial cerebral vascular disease. As with ischemic stroke from arterial disease, the primary mechanism of brain damage is the reduction in capillary blood flow, in this instance because of increased outflow resistance from the blocked veins. Back transmission of high pressure into the capillary bed usually results in early brain swelling from edema and hemorrhagic infarction in subcortical white matter. The most dangerous form of venous disease arises when the superior sagittal sinus is occluded. Venous occlusion occurs in association with coagulation disorders, often in the purpural period, or in subjects with disseminated cancers or contagious diseases. If anticoagulant therapy is not initiated, superior sagittal sinus occlusion has a mortality rate of 25-40%.

Brief diffuse cerebral ischemia can cause syncope without any permanent sequelae. Prolonged diffuse ischemia in other organs have devastating consequences. The most common cause is a cardiac asystole or other cardiopulmonary failures, including infarction. Aortic dissection and global hypoxia or carbon monoxide poisoning can cause similar pictures. Diffuse hypoxia/ischemia typically kills neurons in the hippocampus, cerebellar Purkinje cells, striatum or cortical layers. Clinically, such a diffuse hypoxia/ischemia results in unconsciousness and in coma, followed in many instances by a chronic vegetative state. If the subject does not regain consciousness within a few days, the prognosis for the return of independent brain functions becomes very poor.

Other less common causes of cerebral focal stroke are hematologic diseases associated with thromboses. Such disorders include deficiencies of antithrombin III, protein C, or protein S; polycythemia vera; sickle cell anemia; disseminated intravascular coagulation; and thrombotic thrombocytopenic purpura (TTP). Exogenous agents such as oral contraceptives and e-aminocaproic acid (EACA), drugs such as amphetamines and cocaine, and metabolic disorders, such as homocystinuria, have also been associated with cerebral thrombosis.

The eventual extent of neurologic recovery depends on the patient's age and general state of health as well as on the site and size of the infarction. Impaired consciousness, mental deterioration, aphasia, or severe brainstem signs all suggest a poor prognosis. Complete recovery is uncommon, but the sooner improvement begins, the better the prognosis. About 50% of patients with moderate or severe hemiplegia, and most of those with lesser deficits, recover functionally by the time of discharge and are ultimately able to care for their basic needs, have a clear sensorium, and can walk adequately, although use of an affected limb may be limited. Any deficit remaining after 6 months is likely to be permanent, although some patients continue to improve slowly. Recurrence of cerebral infarction is relatively common, and each recurrence is likely to add to the neurologic disability.

### Assays

The brain is only about 2% of the total body mass, yet it consumes approximately 20% of all the inspired oxygen. Although neurons depend on oxidative metabolism for survival, a consequence of this process is the production of reactive compounds such as hydrogen peroxide and oxy radicals (Cohen and Werner, 1994). In spite of the high vulnerability of the brain to oxygen radical attack, oxygen free-radical reactions and oxidative damage are in most cases held in check by antioxidant defense mechanisms under basal conditions. Pathological conditions of the central nervous system exist, however, where excessive amounts of oxygen free radicals are produced that impair defense mechanisms. Unchecked, these reactive oxygen species (ROS) can lead to DNA damage, peroxidation of membrane lipids and neuronal death.

Oxidative damage caused by free radical production and lipid peroxidation as well as by products of the arachidonic acid cascade are considered to be primary factors in the acute stage pathology of ischemia. Increases in the amounts of free fatty acids after ischemia and during early reperfusion can provide the substrate for lipid peroxidation and for the formation of products of the arachidonic acid cascade (Clemens, et al., Stroke, Vol. 22, No. 8, Aug. 1991).

As discussed above, an ischemic stroke may result from either embolic or thrombotic occlusion of an intracerebral artery, a distinction that is often difficult to make on clinical grounds. Nearly 50 percent of patients with stroke have had one or more preminitory TIAs.

The diagnosis of cerebral focal stroke is strongly supported by the presence of conditions that predispose to embolus formation, such as mitral stenosis, atrial fibrillation, endocarditis, or myxomatous mitral valve prolapse. In addition to dislodged thrombi, emboli may also consist of fat, tumor cells, and air or nitrogen bubbles.

Stroke symptoms generally evolve rapidly, often becoming maximal within seconds to minutes. Occasionally, stroke progresses over minutes or hours, and, rarely, stepwise over days or weeks.

Factors leading to the development of cerebral focal stroke fall mainly into two categories:
1. Disorder of cranial blood vessels. By far, the most common cause of cerebral thrombosis is atherosclerosis, very often associated with hypertension, diabetes mellitus, and coronary artery or peripheral vascular disease. Inflammatory blood vessel disorders, which occur in syphilis, tuberculosis, temporal arteritis, or collagen vascular disease or as a result of radiation injury, may also predispose to thrombus formation. Trauma to the head and neck may cause dissection or thrombosis of major arteries.
2. Disorders of the cardiovascular system. Stroke may follow systemic hypotension from myocardial infarction, heart block, shock, surgical anesthesia, or overly vigorous treatment of chronic hypertension.

Stroke usually can be diagnosed clinically, especially in a person over age 50 with hypertension, diabetes mellitus, or signs of atherosclerosis, or in anyone with a known source of emboli. In the unusual case, differentiation from a rapidly growing or suddenly symptomatic tumor is aided by a CT or MRI scan (which is sometimes negative for as long as several days after an acute infarction). Arteriography is limited to patients in whom the diagnosis is in doubt or remedial vascular obstruction is suspected.

A stroke due to a large embolus tends to be an acute completed stroke, sudden in onset, with focal disorders that are maximal within minutes. Headache may precede it. Thrombosis, which is less frequent, is suggested by a slower onset or gradually progressing symptoms (as in evolving stroke). However, the distinction is not entirely reliable. Concomitant signs of MI, atrial fibrillation, or vegetative heart disease further suggest embolization.

Other than aspirin, ticlopidine is the only antiplatelet agent that has been clearly shown to be effective for stroke prevention. Endarterectomy may be indicated in patients with 70 to 99 percent narrowing of a symptomatic internal carotid artery. However, most authorities agree that carotid endarterectomy is not indicated in patients with TIAs that are referable to the basilar-vertebral system, in patients with significant deficits from prior strokes, or in patients in whom a stroke is evolving.

Heparin may stabilize symptoms in evolving stroke, but anticoagulants are useless (and possibly dangerous) in acute completed stroke, and are contraindicated in hypertensives because of the increased possibility of hemorrhage into the brain or other organs. Although the timing is controversial, anticoagulants may be started to prevent recurrent cardiogenic emboli. Clot lysing agents, including tissue plasminogen activator and streptokinase, are being evaluated for the very early treatment of acute stroke. Nimodipine has recently been shown to improve survival and clinical outcome after ischemic stroke.

To be effective, treatments to minimize brain damage from acute stroke have to begin very soon after stroke onset.

The present invention describes the use of olanzapine for the manufacture of a medicament for the prevention and treatment of cerebral focal stroke.

### Assays

### Assay 1:

The effectiveness of Olanzapine to inhibit the binding of tritiated LTB₄ to guinea pig lung membranes is determined as follows.

### [³H]-LTB₄ Radioligand Binding Assay in Guinea Pig Lung Membranes

[³H]-LTB₄ (196-200 Ci/mmole) was purchased from New England Nuclear (Boston, MA). All other materials were purchased from Sigma (St. Louis, MO). Incubations (555 mL) were performed in polypropylene minitubes for 45 minutes at 30°C and contained 25 mg of guinea pig lung membrane protein (Saussy, et al., Mol. Pharmacol., 39, 72 (1991)) in a buffer containing 25 mM MOPS, 10 mM MgCl₂, 10 mM CaCl₂, pH 6.5, approximately 140 pM [³H]-LTB₄, and displacing ligand or vehicle (0.1% DMSO in 1 mM sodium carbonate, final concentration) as appropriate. The binding reaction was terminated by the addition of 1 mL ice cold wash buffer (25 mM Tris-HCl, pH 7.5) followed immediately by vacuum filtration over Whatman GF/C glass fiber filters using a Brandel (Gaithersburg, MD) 48 place harvester. The filters were washed three times with 1 mL of wash buffer. Retained radioactivity was determined by liquid scintillation counting at 50% counting efficiency using Ready Protein Plus cocktail (Beckman, Fullerton, CA). Nondisplaceable binding is determined in the presence of 1 mM LTB₄ and is usually less than 10% of total binding. Data are analyzed using linear regression analysis of log-log plots of the values between 10% and 90% of control binding to calculate IC₅₀s and slope factors (pseudo-Hill coefficients). IC₅₀ values thus obtained are corrected for radioligand concentration (Cheng and Prusoff, Biochem. Pharmacol., 22, 3099 (1973)) to calculate Kᵢ values. pKi is the mean -log Kᵢ for n experiments.

Olanzapine is tested in the above assay to establish the pKi.

The ability of a Olanzapine to limit effectively neuronal tissue damage can be evaluated in a variety of experimental stroke models (Hunter, el al., TiPS 16, 123-8, 1995). The methodology for testing the compound's effect in ameliorating ischemic cell damage after transient middle cerebral artery occlusion (Zhang, et al., Stroke 26, 1438-43, 1995) is given as one example.

### Assay 2

Male rats weighing approximately 300 grams are anesthetized with 3.5% halothane and maintained with 1.0% halothane in 70% N₂O and 30% O₂ with the use of a face mask. Rectal temperature is maintained at 37°C throughout the surgical procedure. The right femoral artery and vein are cannulated for measuring blood gases before inducing ischemia, monitoring blood pressure during the surgery and administering compound.

Approximately 18 mm of 4-0 surgical nylon suture with a rounded tip is advanced from the external carotid artery into the lumen of the internal carotid artery until it blocks the origin of the middle cerebral artery (MCA). Two hours after MCA occlusion animals are reanesthetized and reperfusion allowed to occur by withdrawing the filament until the tip becomes visible at the origin of the lumen of the external carotid artery. Compound is given by infusing it intravenously over a 3-minute interval at 1, 11, and 22 hours after reperfusion is initiated. Animals are killed 48 hours after reperfusion by giving them ketamine (44 mg/kg) IM and xylazine (13 mg/kg IM). Each rat is transcardially perfused with heparinized saline and 10% formalin before removing the brain. The brain tissue is processed, embedded in paraffin and 6-µm-thick sections cut from each block and stained with hematoxylin and eosin. Infarct volume is measured blindly with the use of a Global Lab Image analysis program (Data Translation). The infarct area and the ipsilateral and contralateral hemispheric area are calculated in square millimeters by tracing the right and left hemispheres and the ischemic lesion on the computer screen. The volumes in cubic millimeters are determined by multiplying the area by the section interval thickness. Infarct volume is analyzed as a percentage of the whole hemisphere. To determine dose-response effects, animals are divided into 5 experimental groups of 10 rats each. The groups are: sham-treated rats given vehicle, stroke-impaired rats given either vehicle or doses of either 0.5, 1.0, 2.0, or 10 mg/kg of olanzapine in saline. The effectiveness of a treatment is accessed by comparing the size of the infarct of the treated group to that of the vehicle control.

The therapeutic and prophylactic treatments provided by this invention are practiced by administering to a mammal in need thereof a dose of olanzapine or a pharmaceutically acceptable salt or solvate thereof, that is effective to inhibit or treat cerebral focal stroke.

The term "inhibit" includes its generally accepted meaning which includes prohibiting, preventing, restraining and slowing, stopping or reversing progression, severity or a resultant symptom. As such, the present method includes both medical therapeutic and/or prophylactic administration as appropriate.

### Assay 3

Cerebral ischemia is produced in rats by occluding the four arteries that supply blood to the brain according to the following procedure. Male Wistar rats are anesthetized with Metofane and placed into a stereotaxic instrument. A longitudinal incision is made on the dorsal surface of the neck. The neck muscles are reflected to expose the dorsal surface of the spinal column. The two vertebral arteries are exposed where they pass through the first cervical vertebra. Both arteries are permanently occluded by the application of electrocautery. After coagulation of the vertebral arteries, the rat is removed from the stereotaxic instrument and the surgical wound is sutured. Two longitudinal incisions are then made on the ventral surface of the neck. The two common carotid arteries are exposed and dissected free from surrounding nerves and connective tissue. An atraumatic clasp, fabricated mainly from silicone rubber tubing, is placed around each carotid artery in a manner such that the vessel is not traumatized or occluded. An indwelling jugular cannula is implanted into each rat for drug delivery. The surgical wounds are then closed. The atraumatic clasps were designed in such a manner that they could be tightened to occlude the carotid arteries by pulling on a small silastic thread that is allowed to protrude from the wound. Circulation to the brain through the carotids could be restored by relieving the tension on the silastic threads. After the surgery, the rats are allowed to recover for 24 hours.

Cerebral ischemia is induced by tightening the clasps around the carotids. During this time, rats in which ischemia is successfully produced lose the righting reflex and became unresponsive to stimuli. The period of ischemia is 20 minutes, and immediately after the 20 minutes of ischemia, at the time of reperfusion, compounds are administered as an intravenous bolus injection of 10 mg/kg followed by a constant intravenous infusion of 5.0 mg/kg per hour for 20 hours. Five days after the ischemia, the rats are sacrificed, and the brains are perfused, fixed with formalin and processed for histological evaluation.

One of the areas of the brain that is most susceptible to ischemia induced damage both in the rat and the human is the CA₁ pyramidal cell layer of the hippocampus. In animals that remain unresponsive for the 20 minute period of ischemia, the CA₁ pyramidal cell layer is completely destroyed. This layer of cells was examined microscopically in histological sections prepared from the hippocampus. Brain damage was rated according to the following scale:
0 = no damage, completely intact cell layer
1 = mild damage, one-third of CA₁ layer dead
2 = moderate damage, two-thirds of CA₁ layer dead
3 = severe damage, greater than 90% cell death

Damage in 4 sections of the dorsal hippocampus from each brain is assessed in order to obtain an accurate estimate of damage. An average damage score is calculated for each treatment group. Scores from treated groups are compared statistically with scores from control groups which received only the vehicle (phosphate buffered saline) that was used to suspend the compounds. The level of significance is determined using the Mann Whitney-U-test.

Olanzapine is tested in the above-described assays to illustrate the usefulness for the claimed methods.

### Pharmaceutical Formulations

As noted above, olanzapine is capable of slowing cerebral focal stroke damage thereby lending itself to the valuable therapeutic methods claimed herein. This method comprises administering to a mammal in need of treatment for cerebral focal stroke an amount of olanzapine effective in achieving the therapeutic effect desired.

Olanzapine utilized in the method of the present invention is effective over a wide dosage range for the treatment of cerebral focal stroke. Thus, as used herein, the term "therapeutically effective amount" refers to a dosage range of from 0.5 to 50 mg/kg of body weight per day. In the treatment of adult humans, the range of 1.0 to 25 mg/kg per day, is preferred. However, it will be understood that the amount of the compound actually administered will be determined by a physician, in the light of the relevant circumstances including the choice of compound to be administered, the chosen route of administration, the age, weight, and response of the individual patient, and the severity of the patient's symptoms, and therefore the above dosage ranges are not intended to limit the scope of the invention in any way.

Ischemia represents a phenomenon in which tissue is deprived of either partial or total blood flow in conjunction with hypoxia. It may occur as an acute event or a chronic condition. The term "acute" means an exacerbated condition of short course followed by a period of remission. Thus, the treatment of ischemia induced cell damage contemplates both acute and chronic forms. In an acute event, the compound is administered at the onset of symptoms and discontinued when the symptoms disappear. As described above, a chronic condition is treated throughout the course of the disease.

Surprisingly, such experiments demonstrate that olanzapine provides a significant effect in the treatment or inhibition of cerebral focal stroke.

The materials for the present invention can be purchased or prepared by a variety of procedures well known to those of ordinary skill in the art. Olanzapine can be prepared as described by Chakrabarti in U.S. Patent No 5,229,382 ('382), herein incorporated by reference in its entirety. Further, the following preparations illustrate a method for preparing the especially preferred Form II olanzapine polymorph.

Compound characterization methods include, for example, x-ray powder pattern analysis, thermogravimetric analysis (TGA), differential scanning calorimetery (DSC), titrametric analysis for water, and H¹-NMR analysis for solvent content.

The following examples are provided for purposes of illustration and are not to be construed as limiting the . scope of the claimed invention.

### Preparation 1

### Technical Grade olanzapine

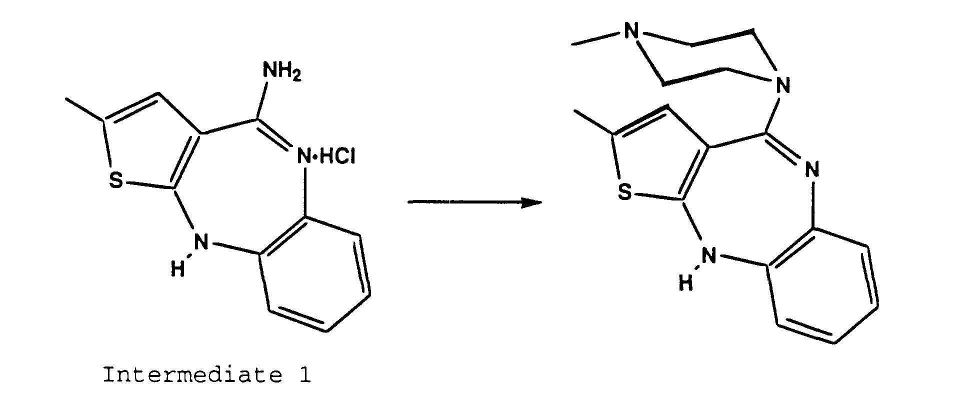

In a suitable three neck flask the following was added:

| | |
|---|---|
| Dimethylsulfoxide (analytical) | 6 volumes |
| Intermediate 1 | 75 g |
| N-Methylpiperazine (reagent) | 6 equivalents |

Intermediate 1 can be prepared using methods known to the skilled artisan. For example, the preparation of the Intermediate 1 is taught in the '382 patent.

A sub-surface nitrogen sparge line was added to remove the ammonia formed during the reaction. The reaction was heated to 120°C and maintained at that temperature throughout the duration of the reaction. The reactions were followed by HPLC until ² 5% of the intermediate 1 was left unreacted. After the reaction was complete, the mixture was allowed to cool slowly to 20°C (about 2 hours). The reaction mixture was then transferred to an appropriate three neck round bottom flask and water bath. To this solution with agitation was added 10 volumes reagent grade methanol and the reaction was stirred at 20°C for 30 minutes. Three volumes of water was added slowly over about 30 minutes.
The reaction slurry was cooled to zero to 5°C and stirred for 30 minutes. The product was filtered and the wet cake was washed with chilled methanol. The wet cake was dried in vacuo at 45°C overnight. The product was identified as technical olanzapine.
Yield: 76.7%; Potency: 98.1%

### Preparation 2

### Form II olanzapine polymorph

A 270 g sample of technical grade 2-methyl-4-(4-methyl-1-piperazinyl)-10H-thieno[2,3-b] [1,5]benzodiazepine was suspended in anhydrous ethyl acetate (2.7 L) . The mixture was heated to 76°C and maintained at 76°C for 30 minutes. The mixture was allowed to cool to 25°C. The resulting product was isolated using vacuum filtration. The product was identified as Form II using x-ray powder analysis.
Yield: 197 g.

The process described above for preparing Form II provides a pharmaceutically elegant product having potency ≥ 97%, total related substances < 0.5% and an isolated yield of > 73%.

### EXAMPLE 1

A portion of the hydroxypropyl cellulose was dissolved in purified water to form a solution for granulation. The remaining hydroxypropyl cellulose (total of 4.0% w/w final tablet weight), which was an extra fine grade, was combined with the olanzapine (1.18% w/w), lactose (79.32% w/w) and a portion of the crospovidone (5% w/w) in a high shear granulator. All ingredients were security sieved prior to addition and dry blended in the granulator. This mixture was then granulated with the hydroxypropyl cellulose solution in the high shear granulator. The granulation was wet sized using standard methods. The wet granulation was then dried in a fluidized bed dryer and sized. The material was then added to a tumble bin mixer.

The running powders consisting of microcrystalline cellulose (granular) (10% w/w), magnesium stearate (0.5% w/w), and the remainder of the crospovidone were added to the sized granulation. The mixture was blended and compressed with the appropriate tooling on tablet compression equipment.

### Subcoating:

Hydroxypropyl methylcellulose (10% w/w) was mixed with purified water to form a solution. Core tablets were divided into approximately equal sections and spray coated with the hydroxypropyl methylcellulose solution . The operation was performed in a perforated coating pan.

### Coating of Core Tablets:

Color Mixture White (hydroxypropyl methylcellulose, polyethylene glycol, polysorbate 80, and titanium dioxide) was mixed with purified water to form the coating suspension. Subcoated tablets were divided into approximately equal sections and spray coated with the coating suspension described above. The operation was performed in a perforated coating pan.

The coated tablets were lightly dusted with carnauba wax and imprinted with appropriate identification.

## Claims

1. Use of olanzapine for the manufacture of a medicament for preventing or treating cerebral focal ischemia in a mammal.

2. Use of **Claim 1** wherein olanzapine is Form II olanzapine polymorph having a typical x-ray diffraction pattern as follows, wherein d represents the interplanar spacing:
| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

3. Use of **Claim 1** wherein the dosage of olanzapine is from 1 mg to 30 mg per day.

4. Use of **Claim 1** wherein the mammal is treated for cerebral focal stroke.

## Patentansprüche

1. Verwendung von Olanzapin zur Herstellung eines Arzneimittels zur Prävention oder Behandlung der cerebralen fokalen Ischämie bei einem Säuger.

2. Verwendung nach Anspruch 1, worin Olanzapin ein Form II Olanzapinpolymorph mit dem folgenden typischen Röntgenbeugungsmuster ist, worin d für den Interplanarabstand steht:
| d |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007 |

3. Verwendung nach Anspruch 1, worin die Dosierung von Olanzapin 1 mg bis 30 mg pro Tag beträgt.

4. Verwendung nach Anspruch 1, worin der Säuger wegen cerebralem fokalem Schlaganfall behandelt wird.

## Revendications

1. Utilisation d'olanzapine pour la préparation d'un médicament pour prévenir ou traiter une ischémie cérébrale focale chez un mammifère.

2. Utilisation selon la revendication 1, dans laquelle l'olanzapine est le polymorphe d'olanzapine de Forme II ayant un diagramme de diffraction typique des rayons X de la poudre comme suit, dans lequel d représente l'espacement interplanaire :
| d |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007 |

3. Utilisation selon la revendication 1, dans laquelle le dosage de l'olanzapine vaut de 1 mg à 30 mg par jour.

4. Utilisation selon la revendication 1, dans laquelle le mammifère est traité pour cause d'une attaque focale cérébrale.
